# EUROPEAN PATENT APPLICATION

(11) **EP 2 522 334 A1**
(43) Date of publication of application: **14.11.2012**
(21) Application number: 11165475.2
(22) Date of filing: 10.05.2011
(51) Int. Cl.: A61K 8/49, A61Q 7/02, A61P 17/00

(54) **Cosmetic compositions comprising diterpenoic acids, e.g. gibberellic acids**

(71) Applicant: Cutech S.R.L., 35127 Padova (IT)
(72) Inventor: Pertile, Paolo, S. Pietro Viminario (Padova) (IT); Massironi, Michele, Padova (IT)
(74) Representative: Fabry, Bernd

(57) **Abstract**

Suggested are new cosmetic compositions comprising diterpenoic acids.

## Description

### Field of invention

The present invention relates to the area of cosmetics, in particular hair cosmetics, and refers to compositions comprising diterpenoic acids and their use for temporary removal and/or reduction of body or facial hair. The invention also provides a medicament for the prophylaxes and treatment of hirsutism.

### State of the art

It is well known from the state of the art that the life of a hair follicle is characterized by a continuous and cyclical transition between a growth stage of the follicle (anagen) in which, among other things, the development of the hair is observed (by virtue of the activity of the keratinocytes), a subsequent regression stage (catagen) in which the programmed death (apoptosis) of a considerable portion of the cells of the follicle takes place, and a third, quiescence stage (telogen) at the end of which the hair follicle returns to the anagen stage with the formation of a new hair shaft.

The duration of the various stages of the life cycle of a hair follicle substantially depends on its position on the body. For example, while in the scalp region anagen lasts from two to eight years, compared with a period of a few weeks for the catagen stage and a few months for the telogen stage, in the eyebrow region the anagen stage only lasts for a few months. This time ratio also determines the percentage of hair follicles which are present, on average, in the various stages of the cycle, for each region of the body. The durations of the various stages of the cycle, as well as the transition between one stage and another, are regulated by complex biological interactions, the mechanisms of which are not entirely understood, between the various parts of the hair follicle and between the follicle and the surrounding epithelial environment. It is, however, known that these stages are affected by many endogenous and exogenous factors which act, directly or indirectly, on the hair follicle to lengthen or shorten the duration of each individual stage.

Many attempts have been made to identify factors causing an early entry into the catagen phase or disorders of the hair follicle and to provide actives for fighting these symptoms, however, with little success so far. On the other hand, also unwanted hair represents a relevant cosmetic issue and the disclosure of new non-toxic agents inhibiting hair growth would find relevant applications.

The presence of hair in some body regions can represent a relevant aesthetic problem, especially for women. Bleaching, lightening or the removal of hair from lips, legs or arm pits, for instance, represent very common cosmetic treatments devoted to emphasize the typical feminine traits. In other cases, however, an excessive presence of hair can represent a pathologic condition. Usually, only vellus hair is present on several body regions of women, for example, on the lips, chin, chest, etc. It can happen that some women grow coarse, dark hairs in these areas, assuming in this regard some traits that are typical for men. This condition is called hirsutism.

Several methods can be adopted in order to obtain a temporary hair removal, each of them presenting pros and cons:
- Shaving is the simpler and more economic method to remove unwanted hair. It does not cause more hair to grow, but sometimes the hair may look thicker and the growth of new hair becomes immediately evident;
- Plucking and waxing can provide a more persistent removal of hair. Despite the fact that the procedures are fairly safe, they can be painful and, moreover, secondary problems may occur on the treated skin such as, for example, scarring, swelling, and skin darkening;
- Chemicals are also commonly used, but also in this case unfavourable effects can occur on the treated skin such as, for example, inflammation or irritation;
- Laser hair removal (selective photothermolysis) uses a specific wavelength of light and pulse duration to permanently damage individual hair follicles, so that they do not grow back. The method exploits the high absorbance of laser energy performed by dark matter. Consequently, this treatment selectively affects dark targets such as moles and hair follicles with negligible consequences on the surrounding tissue. The method is expensive, and multiple treatments are needed. Furthermore, in spite of theoretical preambles, swelling, scarring, and redness of the skin may occur.

The present invention relates to this problem. More particularly, the concrete problem underlying the present invention has been to slow down the growth of hair follicles and, at the same time, to induce an early decline of the hair follicle to the catagen phase. This hair cycle modulation reduces the time by which the hair follicle produces a new hair shaft while prolonging the phase during which the follicle remains unproductive. Therefore, the present invention is not solely directed at a temporary removal or reduction of body or facial hair, serving a true cosmetic effect, but also at providing a medicament which is useful in the prophylaxes and treatment of diseases associated with unwanted growth of body and facial hair, in particular hirsutism.

### Description of the invention

The object of the present invention are cosmetic compositions, comprising diterpenoic acids, in particular selected from the group consisting of gibberellic acids.

Surprisingly it has been found that compositions containing diterpenoic acids, and in particular gibberellic acids (also called "gibberellins"), inhibit the growth of the hair shaft, declining the hair follicle into the catagen phase. The experimental work performed has clearly shown the effects of these compounds on the hair follicle metabolism and in particular on the dermopapilla modifications, which lead the general metabolism of the follicle organ. These data disclose a completely new knowledge on the activity of gibberellins on the hair follicles. The more so, since the prior art on this topic was indeed misled by the experiments performed on cultures of keratinocytes and other cell types. Despite the stimulating action on cell proliferation including keratinocytes, the hair follicle cycle is negatively affected in this regard, and the production of new hair shaft is depressed. These effects cannot only be used for cosmetic purposes, but also serve as a proof that the compositions are useful in the prophylaxes and treatment of diseases such as, in particular, hirsutism.

### Diterpenoic acids

Among the diterpenoic acids, gibberellic acids (also called gibberellins or GAs) are the most useful species.

Gibberellins are plant hormones involved in the regulation of several developmental processes. Among the most relevant activities, GAs modulate plastic processes such as, for example, the stem elongation and germination as well as life cycle phases such as dormancy, flowering, sex expression, leaf and fruit senescence. These hormones are synthesised into plastids, but further biochemical modifications involving enzymes of other cell organelles and solved in the cytosol are required before they achieve their biologically-active form. The gibberellins are named GA1....GAn in the order of discovery. Gibberellic acid, which was the first gibberellin to be structurally characterised, is GA3.

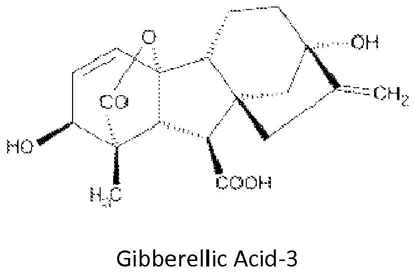

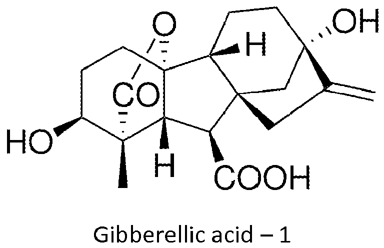

All GAs are characterized by the presence of one of the following groups:
- a tetracyclic (four-ringed) ent-gibberellane skeleton containing 20 carbon atoms (Figure below), or
- a 20-nor-ent-gibberellane skeleton, which has lost the carbon-20 (C-20) remaining with only 19 carbon atoms.

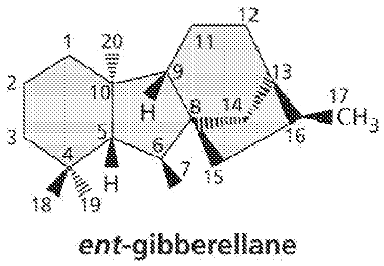

The use of these plant hormones for the treatment of human tissues is limited to few applications, part of which are quite recent.

The first human body treatments were proposed in the early 1980s. In 1981, EP 0079074 A2 disclosed the activity of the gibberellins (used in combination with an optional proteolytic enzyme) for the prevention of hair loss and dandruff, as well as a promoter of hair sprouting and hair growth stimulators. The same Japanese inventor proposed preparations containing different species of gibberellins for dulling the hue of pigments deposited on the skin (JP 59-005105 A2) in the following year.

Other formulations for fighting alopecia were disclosed in 1993 (WO 2004 026240 A1), while the activity of these compounds on the skin cells, with particular reference to keratinocytes and fibroblasts, was taught by some patent applications filed since 1995. EP 0800388 A1 claimed the stimulant activity of GAs on skin cell proliferation and as a hair growth promoter. The same application proposed the use of GAs for improving wound and ulcer healing processes. In 1998, EP 1123083 A1 included the GAs between the compounds cooperating with carotenoids, polyphenols and others in the protection of skin from damages due to UV exposition.

In 2002, EP 1581252 A1 and EP 1587505 B1 claimed the use of GAs in combination with salicylic acid, optionally in combination also with jasmonic acid or zeatin, for stimulating the proliferation of fibroblasts or keratinocytes in the skin and/or stimulating the production of collagen and fibronectin by fibroblasts. The treatments are mainly proposed for the anti-ageing of skin and to promote wound healing, however, hair growth stimulation is also considered among the described effects of these treatments. However, this teaching is basically proposed as a theoretical consequence of the keratinocyte proliferation documented on cell line cultures, while no experimental data were obtained from ex-vivo or in-vivo hair follicles.

French patent FR 2837386 B1 disclosed the stimulation of the cell proliferation use as a consequence of treatments with brown-algae extracts containing GAs.

Among other applications, WO 2000 018069 A1 disclosed the use of GAs and their derivatives for the preparation of a pharmaceutical composition or medicaments for the treatment of diabetes, its complications and associated conditions, including obesity, micro and macro vascular diseases, nephropathy, neuropathy, eye diseases, diabetic ulcerations and similar conditions.

### Cosmetic compositions

The cosmetic compositions according to the present invention are preferably applied topically to the skin where the removal or reduction of body or facial hair is desired. Typically, the diterpenoid acids, and in particular the gibberellic acids are formulated together with cosmetically acceptable carriers such as, for example, water, C₂-C₄ alcohols, polyhydric alcohols and oil components.

### Alcohols and polyhydric alcohols

The compositions may represent aqueous solutions. However, also ethanol, isopropyl alcohol or polyhydric alcohols ("polyols"), may be used as cosmetically acceptable carriers. Suitable polyols preferably contain 2 to 15 carbon atoms and at least two hydroxyl groups. The polyols may contain other functional groups, more especially amino groups, or may be modified with nitrogen. Typical examples are
o glycerol;
o alkylene glycols such as, for example, ethylene glycol, diethylene glycol, propylene glycol, butylene glycol, hexylene glycol and polyethylene glycols with an average molecular weight from 100 to 1000 Dalton;
o technical oligoglycerol mixtures with a degree of self-condensation from 1.5 to 10, such as, for example, technical diglycerol mixtures with a diglycerol content from 40 to 50% by weight;
o methylol compounds such as, in particular, trimethylol ethane, trimethylol propane, trimethylol butane, pentaerythritol and dipentaerythritol;
o lower alkyl glucosides, particularly those containing 1 to 8 carbon atoms in the alkyl group, for example, methyl and butyl glucoside;
o sugar alcohols containing 5 to 12 carbon atoms, for example, sorbitol or mannitol,
o sugars containing 5 to 12 carbon atoms, for example, glucose or sucrose;
o amino sugars, for example, glucamine;
o dialcoholamines, such as diethanolamine or 2-aminopropane-1,3-diol.

### Oil bodies

Suitable oil bodies forming cosmetically acceptable carriers are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C ₁₃-carboxylic acids with linear or branched C₆-C₂₂-fatty alcohols such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆ -C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group such as, for example, dicaprylyl ether (Cetiol^{®} OE), ring-opening products of epoxidized fatty acid esters with polyols, silicone oils (cyclomethicones, silicone methicone grades, etc.), aliphatic or naphthenic hydrocarbons such as, for example, squalane, squalene or dialkylcyclohexanes, and/or mineral oils.

Usually the cosmetic compositions contain from about 0.1 to about 15, preferably from about 1 to about 10, and more preferably from about 2 to about 5 % b.w. diterpenoic acids, preferably gibberellic acids, while the remaining part represents the carrier.

The administration of the diterpenoic acids can be oral, however, a topical administration is preferred. In case of topical application all kinds of compositions are possible: lotions, creams, emulsions and the like. For oral uptake, capsules are the preferred galenic forms. These embodiments are explained in more detail below.

### Capsules and Microcapsules

For oral uptake, the encapsulation of the compositions represents a preferred embodiment. Usually encapsulation can take place by using gelatine as a matrix. It is also possible to prepare capsules by adding a gelling agent such as, for example, alginate to the diterpenoic acids composition and to drop the mixture into a bath of a calcium salt. Both methods lead to macro-capsules having a diameter of from about 1 cm to about 5 cm which are toxicologically safe and suitable for consumption.

It may also be advantageous to encapsulate the diterpenoic acids for the formulation of compositions which are developed for topical application. This can have different reasons: stabilisation against an interaction with other compounds in the formulation, protection against chemical degradation, or simply for the preparation of a very aesthetical product. Microcapsules are usually applied for this purpose. "Microcapsules" are understood to be spherical aggregates with a diameter of from about 0.1 to about 5 mm, which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("microsponge") and, as microparticles, may be additionally coated with film-forming polymers. The microscopically small capsules, also known as nanocapsules, can be dried in the same way as powders. Besides single-core microcapsules, there are also multiple-core aggregates also known as microspheres, which contain two or more cores distributed in the continuous membrane material. In addition, single-core or multiple-core microcapsules may be surrounded by an additional second, third, etc. membrane. The membrane may consist of natural, semisynthetic or synthetic materials. Natural membrane materials are, for example, gum arabic, agar agar, agarose, maltodextrins, alginic acid and salts thereof, for example, sodium or calcium alginate, fats and fatty acids, cetyl alcohol, collagen, chitosan, lecithins, gelatin, albumin, shellac, polysaccharides such as starch or dextran, polypeptides, protein hydrolyzates, sucrose and waxes. Semisynthetic membrane materials are, inter alia, chemically modified celluloses, more particularly cellulose esters and ethers, for example, cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers such as polyacrylates, polyamides, polyvinyl alcohol or polyvinyl pyrrolidone. Examples of known microcapsules are the following commercial products (the membrane material is shown in brackets) Hallcrest Microcapsules (gelatin, gum arabic), Coletica Thalaspheres (maritime collagen), Lipotec Millicapseln (alginic acid, agar agar), Induchem Unispheres (lactose, microcrystalline cellulose, hydroxypropylmethyl cellulose), Unicetin C30 (lactose, microcrystalline cellulose, hydroxypropylmethyl cellulose), Kobo Glycospheres (modified starch, fatty acid esters, phospholipids), Softspheres (modified agar agar) and Kuhs Probiol Nanospheres (phospholipids).

### Additives for cosmetic compositions

The cosmetic compositions according to the present invention, preferably compositions for the treatment of human hair, may contain additional compounds such as, for example, surfactants, oil bodies, emulsifiers, superfatting agents, pearlising waxes, consistency factors, polymers, silicone compounds, waxes, stabilizers, antidandruff agents, biogenic agents, film formers, preservatives, perfume oils, dyes and the like as additional auxiliaries and additives.

### Surfactants

Other preferred auxiliaries and additives are anionic and/or amphoteric or zwitterionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl (ether) phosphates. If the anionic surfactants contain polyglycol ether chains, they may have a conventional homologue distribution although they preferably have a narrow-range homologue distribution. Typical examples of amphoteric or zwitterionic surfactants are alkylbetaines, alkylamidobetaines, aminopropionates, aminoglycinates, imidazolinium betaines and sulfobetaines. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 **or** J. Falbe (ed.), "Katalysatoren, Tenside und Mineralöladditive (Catalysts, Surfactants and Mineral Oil Additives)", Thieme Verlag, Stuttgart, 1978, pages 123-217**.** The percentage content of surfactants in the preparations may be from 0.1 to 10% by weight and is preferably from 0.5 to 5% by weight, based on the preparation.

### Oil bodies

Suitable oil bodies are those which have already been compiled among the suitable cosmetic carriers.

### Emulsifiers

Other surfactants may also be added to the preparations as emulsifiers including, for example:
o products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
o C_{12/18} fatty acid monoesters and diesters of addition products of 1 to 30 mol ethylene oxide onto glycerol;
o glycerol mono- and diesters and sorbitan mono- and diesters of saturated and unsaturated fatty acids containing 6 to 22 carbon atoms and ethylene oxide addition products thereof;
o addition products of 15 to 60 mol ethylene oxide onto castor oil and/or hydrogenated castor oil;
o polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
o addition products of 2 to 15 mol ethylene oxide onto castor oil and/or hydrogenated castor oil;
o partial esters based on linear, branched, unsaturated or saturated C_{6/22} fatty acids, ricinoleic acid and 12-hydroxystearic acid and glycerol, polyglycerol, pentaerythritol, -dipentaerythritol, sugar alcohols (for example, sorbitol), alkyl glucosides (for example, methyl glucoside, butyl glucoside, lauryl glucoside) and polyglucosides (for example, cellulose);
o mono-, di and trialkyl phosphates and mono-, di- and/or tri-PEG-alkyl phosphates and salts thereof;
o wool wax alcohols;
o polysiloxane/polyalkyl polyether copolymers and corresponding derivatives;
o mixed esters of pentaerythritol, fatty acids, citric acid and fatty alcohol and/or mixed esters of C₆₋₂₂ fatty acids, methyl glucose and polyols, preferably glycerol or polyglycerol,
o polyalkylene glycols and
o glycerol carbonate.

The addition products of ethylene oxide and/or propylene oxide onto fatty alcohols, fatty acids, alkylphenols, glycerol mono- and diesters and sorbitan mono- and diesters of fatty acids or onto castoroil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations.

Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example. Other suitable emulsifiers are zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### Superfatting agents

Superfatting agents may be selected from substances such as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

### Consistency factors

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners such as Aerosil^{®} types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example, Carbopols^{®} [Goodrich] or Synthalens^{®} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lamequat^{®} L, Grünau), quaternized wheat polypeptides, polyethyleneimine, cationic silicone polymers such as, for example, amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar CBS, Jaguar C-17, Jaguar^{®} C-16 of Celanese, quaternized ammonium salt polymers such as, for example, Mirapol^{®} A-15, Mirapol^{®} AD-1, Mirapol^{®} AZ-1 of Miranol. Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlising waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**).**

### Waxes and Stabilizers

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes. Metal salts of fatty acids such as, for example, magnesium, aluminium and/or zinc stearate or ricinoleate may be used as stabilizers.

### Biogenic agents

In the context of the invention, biogenic agents are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example, prune extract, bambara nut extract, and vitamin complexes.

### Film formers

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

### Anti-dandruff agents

Suitable antidandruff agents are Pirocton Olamin (1-hydroxy-4-methyl-6-(2,4,4-trimethylpentyl)-2-(1H)-pyridinone monoethanolamine salt), Baypival^{®} (Climbazole), Ketoconazol^{®} (4-acetyl-1-{4-[2-(2,4-dichlorophenyl) r-2-(1H-imidazol-1-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon^{®} UD (protein/undecylenic acid condensate), zinc pyrithione, aluminium pyrithione and magnesium pyrithione/dipyrithione magnesium sulfate.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Perfume oils

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example, civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### Dyes

Suitable dyes are any of the substances suitable and approved for cosmetic purposes as listed, for example, in the publication **"**Kosmetische Färbemittel" of the Farbstoffkommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. These dyes are normally used in concentrations of from 0.001 to 0.1% by weight, based on the mixture as a whole.

The total percentage content of auxiliaries and additives may be from 1 to 50 % by weight and is preferably from 5 to 40 % by weight, based on the particular composition. The compositions may be produced by standard hot or cold processes.

### Industrial application

### Non-therapeutic treatment of human hair

Further objects of the present invention refer to the use of diterpenoic acids, preferably gibberellic acids for the treatment of human hair, and, in particular, for the temporary removal and/or reduction of body or facial hair.

Another object of the present invention refers to a non-therapeutic method for the temporary removal and/or reduction of body or facial hair, which is characterised in that a composition comprising a working amount of at least one diterpenoic acid, preferably at least one gibberellic acid, is applied to the skin. Preferably said diterpenoic acids, and more preferably said gibberellic acids, are present in said compositions in amounts of from about 0.001 to about 100, and more preferably in amounts of from about 0.01 to about 10 µg/ml.

Diterpenoic acids in general and gibberellic acids in particular inhibit or at least reduce the growth of hair follicles and anticipate the entry into the catagen phase as demonstrated in the following working examples.

### Therapeutic uses

Further objects of the present invention concern:
(i) a medicament comprising diterpenoic acids, in particular gibberellic acids;
(ii) a medicament comprising diterpenoic acids, in particular gibberellic acids, which is administered topically at a dosage of from about 0.001 to 1000 µg/ml daily over a period of at least 14 and preferably 14 to 28 days;
(iii) a medicament comprising diterpenoic acids, in particular gibberellic acids, for the temporary removal and/or reduction of body and facial hair;
(iv) a medicament comprising diterpenoic acids, in particular gibberellic acids, for the prophylaxes and treatment of hirsutism.

### Examples

### Examples 1 to 3

### Activity of gibberellins on the growth of hair follicles

Human anagen hair follicles were isolated from scalp skin taken from a single donor and transferred for cultivation in sterile 24 well plates using a modified Williams' Medium E. Cultivation took place for nine days, while the experimental treatment of the follicles started 24 hours from the beginning of the cultivation. Hair follicles were selected for the experiments after 18 h of cultivation. Only follicles showing a good vital stage and a growth of not less than 0.2 mm were considered suitable to be maintained in culture. All experimental groups and the control group were prepared by including 12 follicles, plated in 24 well plates at a density of 3 hair follicles (HFs) per well. Hair follicles showing evident signs of sufferance during the culture for reasons that did not depend on the experimental treatment were excluded from the final analysis. The following experiments were conducted to demonstrate the activity of gibberellin (GA3) on hair follicle growth in a concentration of 0.01 to 1 µg/ml compared to a control group. The activity of the gibberellins treatment is demonstrated by the decrease of growth of the hair follicles expressed in percentage of the performance recorded in the control group. The growth of the hair follicles was studied by performing image analyses on microphotographs. All hair follicles were photographed at day 5 of culture (day 4 of treatment) and then at day 9 of culture (day 8 of treatment). The results detected at day 9 of culture are presented in Table 1:

**Table 1**

| Growth of hair follicles: Elongation performance expressed as percentage of the control group | | | | | |
|---|---|---|---|---|---|
| Example | Sample | Conc. [µg ml⁻¹] | Average | Total n. of HFs | ANOVA |
| 0 | Control | 0 | 100.0 ± 4.6 | 18 | |
| 1 | Gibberellin | 0.01 | 88.2 ±7.0 | 10 | n.s. |
| 2 | Gibberellin | 0.1 | 85.6 ±5.4 | 12 | P<0.05 |
| 3 | Gibberellin | 1.0 | 80.6 ± 5.4 | 12 | P <0.01 |

The results attest that the addition of gibberellin leads to a significant decrease in the growth of hair follicles. The dose response experiment has shown the increasing action of Gibberellin depending on the tested concentration. When tested at 1 µg/ml, gibberellin has exerted an inhibitory action on hair growth of 19%, in comparison with the untreated sample. This inhibition is very significant on a statistical basis (P<0.01)

### Examples 4 to 6

### Activity of gibberellins on the hair follicles growth

The experiment according to Examples 1 to 3 was terminated after 9 days of cultivation. The results are shown in Table 2:

**Table 2**

| Growth of hair follicles: Elongation performance expressed as percentage of the control group | | | | |
|---|---|---|---|---|
| Example | Sample | Conc. [µg ml⁻¹] | Average | Total n. of Hfs. |
| 0 | Control | 0 | 100.0 ± 6.2 | 14 |
| 4 | Gibberellin | 0.01 | 81.4 ± 5.9 | 10 |
| 5 | Gibberellin | 0.1 | 89.8 ± 8.0 | 11 |
| 6 | Gibberellin | 1.0 | 82.7 ± 6.6 | 10 |

The results indicate that the gibberellins reduce hair growth by 10 to 19 % in comparison to the control group depending on the treatment dosage.

### Examples 7 to 8

### Activity of gibberellins on the growth of hair follicles

The experiment according to Examples 1 to 3 was terminated after 9 days of cultivation. The results are shown in Table 3:

**Table 3**

| Growth of hair follicles: Elongation performance expressed as percentage of the control group | | | | | |
|---|---|---|---|---|---|
| Example | Sample | Conc. [µg ml⁻¹] | Average | Total n. of HFs | ANOVA |
| 0 | Control | 0 | 100.0 ± 6.1 | 14 | |
| 7 | Gibberellin | 0.1 | 76.6 ± 4.3 | 10 | P<0.01 |
| 8 | Gibberellin | 1.0 | 69.9 ± 3.2 | 11 | P< 0.001 |

The results indicate that the gibberellins reduce hair growth by 23 to 30 % in comparison to the control group depending on the treatment dosage. Both treatment results are highly significant on a statistical basis, however, the best response has been detected by treating the follicles with 1 µg/ml (-30.1 % in comparison to the control group; P<0.001).

### Examples 9 to 10

### Activity of gibberellins on the hair follicles growth

The experiment according to Examples 1 to 3 was terminated after 9 days of cultivation. The results are shown in Table 4:

**Table 4**

| Growth of hair follicles: Elongation performance expressed as percentage of the control group | | | | | |
|---|---|---|---|---|---|
| Example | Sample | Conc. [µg ml⁻¹] | Average | Total n. of HFs | ANOVA |
| 0 | Control | 0 | 100.0 ± 4.2 | 17 | |
| 9 | Gibberellin | 0.1 | 77.7 ± 5.8 | 11 | P <0.01 |
| 10 | Gibberellin | 1.0 | 72.3 ± 4.9 | 10 | P< 0.001 |

The results indicate that the gibberellins reduce hair growth by 22 to 28 % in comparison to the control group depending on the treatment dosage. Both treatment results are highly significant on a statistical basis, however, the best response has been detected by treating the follicles with 1 µg/ml (-27.7 % in comparison to the control group; P<0.001).

### Examples 11 to 12

### Activity of gibberellins on the growth of hair follicles

The experiment according to Examples 1 to 3 was terminated after 9 days of cultivation. The results are shown in Table 5:

**Table 5**

| Growth of hair follicles: Elongation performance expressed as percentage of the control group | | | | |
|---|---|---|---|---|
| Example | Sample | Conc. [µg ml⁻¹] | Average | ANOVA |
| 0 | Control | 0 | 100.0 ± 4,6 | |
| 11 | Gibberellin | 0.1 | 91,3 ± 4.7 | n.s. |
| 12 | Gibberellin | 1.0 | 79.9 ± 5.9 | P<0.01 |

The results indicate that the gibberellins reduce hair growth by up to 20% in comparison to the control group depending on the treatment dosage. The treatment at 1 µg/ml shows a result that is highly significant on a statistical basis, (-20% in comparison to the control group; P<0.01).

### Examples 13 to 14

### Activity of gibberellins on the occurrence of the catagen stage of hair follicles

The experiment according to Examples 1 to 3 was terminated after 9 days of cultivation. Subsequently, the hair follicles were subjected to histological analysis by staining with haematossilin and eosin in order to verify the morphological state of the dermopapilla. The results are shown in Table 6:

**Table 6**

| Histological analysis of hair follicles | | | | |
|---|---|---|---|---|
| Example | Sample | Amount | Anagen phase | Catagen phase |
| 0 | Control | 0 | 41.7 | 58.3 |
| 13 | Gibberellin | 0.1 µg/ml | 22.2 | 77.8 |
| 14 | Gibberellin | 1 µg/ml | 9.1 | 90.9 |

The results indicate that the gibberellin treatment has significantly anticipated the decline into the catagen phase of the hair follicles. Since growth of hair follicles only takes place during the anagen phase, these results further contribute to supporting the property of gibberellins in inhibiting hair growth.

## Claims

1. Cosmetic composition comprising diterpenoic acids.

2. Compositions according to Claim 1, **characterised in that** they comprise diterpenoic acids selected from the group consisting of gibberellic acids.

3. Compositions according to Claims 1 and/or 2, **characterised in that** they comprise cosmetically acceptable carriers.

4. Compositions according to any of the preceding Claims 1 to 3, **characterised in that** they comprise cosmetically acceptable carriers selected from the group consisting of water, C₂-C₄ alcohols, polyhydric alcohols and oil components.

5. Use of diterpenoic acids for treatment of human hair.

6. Use of gibberellic acids for treatment of human hair.

7. Use of diterpenoic acids for the temporary removal and/or reduction of body or facial hair.

8. Use of gibberellic acids for the temporary removal and/or reduction of body or facial hair.

9. A non-therapeutical method for the temporary removal and/or reduction of body or facial hair, **characterized in that** a composition comprising a working amount of at least one diterpenoic acid is applied to the skin.

10. A method according to Claim 9, **characterized in that** said diterpenoic acid is a gibberellic acid.

11. A method according to Claims 9 and/or 10, **characterised in that** said diterpenoic acid is present in said compositions in an amount of from 0.01 to 1 µg/ml.

12. Medicament comprising diterpenoic acids.

13. Medicament comprising diterpenoic acids, which is administered topically at a dosage of from about 0.001 to 1000 µg/ml daily over a period of at least 14, and preferably from 14 to 28 days;

14. Medicament comprising diterpenoic acids for the temporary removal and/or reduction of body and facial hair.

15. Medicament comprising diterpenoic acids for the prophylaxes and treatment of hirsutism.
